Europäisches Patentamt

European Patent Office

Office européen des brevets

(19)

(11) EP 0 907 664 B1

(12) EUROPEAN PATENT SPECIFICATION

(45) Date of publication and mention
of the grant of the patent:
**23.08.2000 Bulletin 2000/34**

(51) Int Cl.$^7$: **C08B 37/00**, A61L 15/28,
A61K 47/36, A61K 47/38,
A61K 47/48

(21) Application number: **97928373.6**

(22) Date of filing: **27.06.1997**

(86) International application number:
**PCT/GB97/01726**

(87) International publication number:
**WO 98/00446 (08.01.1998 Gazette 1998/01)**

(54) **OXIDIZED OLIGOSACCHARIDES**

OXYDIERTE OLIGOSACCHARIDE

OLIGOSACCHARIDES OXYDES

(84) Designated Contracting States:
**AT BE CH DE ES FR GB GR IE IT LI LU NL PT SE**

(30) Priority: **28.06.1996 GB 9613683**

(43) Date of publication of application:
**14.04.1999 Bulletin 1999/15**

(73) Proprietor: **Johnson & Johnson Medical Ltd.
Edinburgh EH2 4NH (GB)**

(72) Inventors:
• **HARVEY, Wilson
North Yorkshire BD23 3DW (GB)**
• **SAFERSTEIN, Lowell
NJ 07052 (US)**
• **WISEMAN, David
Dallas, Texas 75248 (US)**
• **WATT, Paul, William
West Yorkshire, BD23 6UN (GB)**
• **LIGHT, Nicholas
North Yorkshire BD23 3RX (GB)**

(74) Representative:
**James, Anthony Christopher W.P. et al
Carpmaels & Ransford
43 Bloomsbury Square
London WC1A 2RA (GB)**

(56) References cited:
**EP-A- 0 526 756          FR-A- 2 035 627
US-A- 2 517 772**

• **CHEMICAL ABSTRACTS, vol. 112, no. 4, 22
January 1990 Columbus, Ohio, US; abstract no.
22627, "Electrochemical oxidation of low
molecular-weight dextran" XP002044298 &
JANKIEWICZ B. ET AL.: CHEM. STOSOW., vol.
32, no. 2, 1988, pages 293-299,**
• **CHEMICAL ABSTRACTS, vol. 122, no. 8, 20
February 1995 Columbus, Ohio, US; abstract no.
84137, "Manufacture of oxidized
oligosaccharides having ability of trapping
metal ions and cleaning or detergent
compositions containing the oligosaccharides
as biodegradable builders" XP002044299 & JP
06 279 504 A (LION CORP.) 4 October 1994**

**Description**

This invention relates to a pharmaceutical composition comprising oxidized oligosaccharides such as oligosaccharides of oxidized regenerated cellulose (ORC). The invention also relates to the use of oxidized oligosaccharides in wound dressings and other medical and pharmaceutical applications, and to methods for preparing oxidized oligosaccharides.

ORC has long been manufactured and used medically to achieve haemostasis, and as a barrier material to prevent adhesions following surgery. A key feature of ORC is that it is absorbable when implanted in the body, whereas cellulose is not. ORC is resorbed by hydrolytic cleavage of the polymer to yield small oligosaccharides which are metabolized and eliminated from the body. Oxidation of cellulose to yield 10 to 21% carboxyl groups by weight of the cellulose allows substantially complete absorption of the material within two to three weeks following implantation.

ORC is manufactured by exposure of cellulose to an oxidizing agent such as dinitrogen tetroxide, as described in US-A-3122479. The physical form of cellulosic material is not critical. For example, the cellulosic film, paper, sponge and cloth may all be oxidized to yield ORC. However, the commercially preferred material is a woven or knitted fabric. ORC in the form of a knitted fabric is available under the Trade Mark SURGICEL for use as an absorbable haemostat, and ORC is also available under the Trade Mark INTERCEED for use as an adhesion barrier.

Polysaccharides other than cellulose may also be oxidized to yield medically useful haemostatic materials. Such other polysaccharides include microbial polysaccharides such as dextran, gellan gum and xanthan gum; polysaccharides derived from plants, for example agar, starch, konjac, carrageenan, guar gum, inulin and pectin; and polysaccharide derivatives such as carboxymethyl cellulose, methylhydroxypropyl cellulose, cellulose acetate, methyl cellulose, and ethyl cellulose.

It has been proposed to combine ORC with other materials for use as wound dressings. For example, US-A-2517772 (Doub *et al*) discloses dressings formed from ORC impregnated with thrombin. However, ORC is significantly acidic. The surface pH of a fully water-saturated piece of ORC fabric may be as low as 1.7. Many proteinaceous agents, such as thrombin, are highly acid-sensitive, and are inactivated immediately on contact with such a matrix. Accordingly, Doub *et al* teach that ORC should be neutralized prior to impregnation with thrombin. Calcium acetate, sodium bicarbonate, ammonia and alcoholic ethylamine are given as examples of suitable neutralizing agents, but Doub *et al* warn that ORC should not be neutralized to such a degree that it loses its fibrous nature when placed in water because of solution or gelling and disintegration.

EP-A-0437095 discloses that the use of aqueous solutions of sodium bicarbonate to neutralize ORC cloth results in a cloth which is partially gelled, distorted from its original size and very weak with little integrity. The tensile strength of the cloth is said to be too low for practical use such as, for example, a haemostat. Similar results are said to be obtained with the use of strongly basic aqueous sodium hydroxide and ammonium hydroxide solutions. EP-A-0437095 accordingly teaches a process for preparing a storage stable, non-irritating and therapeutic neutralized oxidized cellulose product comprising the steps of contacting an acid oxidized cellulose material with an alcohol and water solution of a slightly basic chloride-free salt of a weak acid to elevate the pH of the cellulose material to between 5 and 8; washing the elevated pH cellulose material with alcohol to remove excess salt and water; and drying the cellulose material to remove alcohol.

It has now been found that ORC and other oxidized polysaccharides can be partially hydrolysed under mild alkaline conditions to yield oligosaccharides which have a number of medically useful properties.

Accordingly, the present invention provides a pharmaceutical composition for topical, oral or parenteral administration, comprising an oxidized oligosaccharide having an average molecular weight in the range 1000 to 50000 daltons.

In particular, the oxidized oligosaccharide compositions of the present invention bind therapeutically useful agents, and such bound agents can then be released in high yield. The oxidized oligosaccharide pharmaceutical compositions of the present invention can therefore be used, for example in wound dressings, to deliver such agents to a wound site. The therapeutically useful agents which are bound by oxidized oligosaccharides include pharmaceutically active peptides and proteins, preferably growth factors such as PDGF AB, PDGF BB, TGF-$\beta$1, TGF-$\beta$2, TGF-$\beta$3, basic FGF, acidic FGF and possibly EGF and TGF-$\alpha$.

Without wishing to be bound by any theory, it is thought that the anionic carboxylate groups on the oxidized oligosaccharides complex to cationic amine groups on the peptides and proteins. Complexation to therapeutically active agents having anionic groups can also readily be achieved, for example, by use of polyvalent metal ions such as $Ca^{2+}$ or $Zn^{2+}$ as ionic cross-linking agents.

A further advantage of the pharmaceutical compositions of the present invention is that they may be intimately combined with other materials such as proteins and other polysaccharides (with or without chemical cross-linking) to form compositions having novel properties. For example, oxidized oligosaccharides may be combined with hyaluronic acid, chitosan, or an alginate (particularly sodium alginate, calcium alginate or a mixed sodium/calcium alginate) to form novel haemostatic compositions. Alternatively, composites of oxidized oligosaccharides with other oligosaccharides, polysaccharides or proteins may be used as controlled release matrices for a variety of therapeutic agents such as antiseptics, antibiotics, protein growth factors, anti-inflammatories, analgesics, proteinase inhibitors such as aprotinin or the hydroxamic acids. The oxidized oligosaccharides of the compositions of the present invention may be combined with a desired therapeutic agent while in solution (the therapeutic agent being either in solution in suspension), and the oligosaccharide may then be removed from solution by suitable means, to yield a material in which the therapeutic agent is substantially uniformly distributed. Alternatively, the solvent maybe removed, e.g. by freeze-drying.

Oxidized oligosaccharides may also be cross-linked so as to allow the formation of three-dimensional structures. For example, oxidized oligosaccharides can be dissolved in water to which a very low concentration of pepsin-solubilized collagen is added. If carbodiimide is then added as a cross-linker, the collagen acts as a bridging group between the oligosaccharides, such that a three-dimensional structure can be obtained by freeze drying.

The oxidized oligosaccharides in the compositions of the present invention preferably have a molecular weight of at least 1000 daltons, and generally less than 50000. Most usually, the molecular weight will be less than 5000 to 30000 daltons and preferably in the range 5000 to 25000 daltons. (It will be understood that the oxidized oligosaccharides in the present invention will generally form a mixed population of different sized molecules. Accordingly, references herein to oxidized oligosaccharides having a particular molecular weight range signify that at least 90% by weight of the molecules fall within the specified range.)

In one embodiment, oligosaccharides for use in compositions according to the invention are derived from insoluble oxidized polysaccharides and are of such a molecular weight that they are soluble at neutral and alkaline pH, but insoluble at acid pH. Such oligosaccharides can be readily recovered from solution merely by reducing the pH, so causing them to precipitate. Alernatively, however, oxidized oligosaccharides can be recovered from solution by transferring them to a solution which does not contain any other non-volatile components, and then evaporating the solvent. For example, oxidized oligosaccharides can be isolated using an ion exchange solid phase extraction column (such as a phenyl boronic acid solid phase column, previously activated with methanol and equilibrated with dilute acetic acid), and then eluted with dilute (e.g. 0.1M) ammonium hydroxide solution.

Preferably, the oxidized oligosaccharides used in the compositions of the present invention have a carboxyl content of from 5 to 25% by weight, and more preferably from 8 to 14% by weight. The carboxyl content of the oligosaccharides is determined as follows:

A sample of oxidized oligosaccharide (approximately 0.2g) is dissolved in 0.5M sodium hydroxide (5ml) and a couple of drops of 0.1% phenolphthalein indicator solution are added. The excess sodium hydroxide is back-titrated with 0.1M HCl to the phenolphthalein end point (red to clear). A blank value is determined by titrating 5ml 0.1M sodium hydroxide with 0.1M HCl. The value for carboxyl content is calculated using the equation:

$$C = \frac{4.5 \times (B-S) \times M}{W}$$

wherein:

C = percent carboxyl content
B = volume of standard HCl to titrate blank (ml)
S = volume, of standard HCl to titrate sample (ml)
M = morality of standard HCl
W = dry weight of sample (g)
(4.5 = milliequivalent weight of carboxyl x 100)

The present invention also provides a method of preparing an oxidized cellulose, comprising treating an oxidized cellulose having a molecular weight of at least 50000 (more usually at least 100000, e.g. more than 300 000) with an aqueous alkaline solution at a temperature and for a period of time sufficient to result in partial hydrolysis of said oxidized cellulose, and then recovering the resulting oxidized cellulose from solution, e.g. by adjusting the pH to 7 or less. The alkaline solution is conveniently a solution of an alkali metal hydroxide or bicarbonate, e.g. sodium hydroxide or sodium bicarbonate, although other alkalis (e.g. aqueous ammonium hydroxide) can also be used. It will be understood that the treatment conditions (and particularly the pH) are dependent on the desired molecular weight range for the resulting product. However, appropriate conditions can readily be determined in any particular case by routine experiment. By way of example, oxidized regenerated cellulose may be hydrolyzed in 1M to 8M sodium hydroxide at a temperature of from 0°C to 50°C for 5 to 120 minutes to yield oligosaccharides in the molecular weight range 1000 to 20000 daltons, or with 0.01M to 1M sodium bicarbonate at 0°C to 50°C for 10 hours to 10 days to yield oligosaccharides in the molecular weight range 7000 to 50000.

The hydrolytic reaction can be stopped by the addition of an acid, such as a mineral acid, until the solution is approximately neutral. Concentrated hydrochloric acid can conveniently be used.

The invention is further described by the following Examples.

Example 1

A solution of ORC was prepared by dissolving Surgicel™ fabric at a concentration of 20mg/ml in 6M sodium hydroxide.
The solution was incubated at 37°C for 45 minutes after which the reaction was stopped by adding 6M HCl until precipitation occurred and the pH changed from alkaline to pH7 or less. The precipitate was allowed to settle overnight, and then the excess liquid was removed. The precipitate was dialysed against water in tubing with a 1000 molecular weight cut off, then freeze dried to product a powder.
The molecular size of the oligosaccharide, determined by gel electrophoresis and by high performance liquid chromatography, showed a range extending from approximately 1000 to 15000 daltons.

Example 2

A solution of ORC was prepared by dissolving Surgicel™ fabric at a concentration of 10mg/ml in 0.1M sodium bicarbonate. The solution was incubated at 37°C for a few days (2-3) until all the ORC has dissolved. The reaction was stopped by adding 6M HCl until precipitation occurred and the pH changed from alkaline to pH7 or less. The precipitate was allowed to settle overnight and then the excess liquid removed. The precipitate was dialysed against water in tubing with a 1000 molecular weight cut off, then freeze dried to product a powder.
The molecular size, determined as described above, showed a range from approximately 1000 to 30000 daltons.

Example 3

Oxidized carboxymethyl cellulose sponge was prepared as follows:
Into 500 grams of water is added with stirring 7.5 grams of carboxymethylcellulose (CMC) from Aqualon Corporation. When the polymer is dissolved the solution is allowed to deaerate overnight to remove trapped air bubbles. The solution is poured into trays 3x4x¼ inch, and freeze dried for 24 hours in a lyophilizer. Soft, white, water soluble CMC sponges are obtained from this procedure.
The oxidation of the CMC sponges is accomplished by placing 5.8 grams of dry sponges into a resin kettle to which is attached a small flask containing 8 grams of nitrogen tetroxide. The nitrogen tetroxide is allowed to evaporate from the small flask into the resin kettle and envelope the CMC sponges in an atmosphere of gas. The sponges are kept in the resin kettle for 48 hours after which time the gas is evacuated to caustic trap and the sponges are removed and placed in 500ml of water. The oxidized CMC sponges are not soluble in water. They are washed with water for 15 minutes then placed in fresh water for another wash. This washing of the oxidized sponges is repeated until the pH of the wash water is above 3. The white oxidized carboxymethylcellulose sponges are dried by placing them in 100% isopropyl alcohol for 15 minutes. This is repeated for a total of 2 washes then the sponges are allowed to air dry. The oxidized CMC sponges are soft and conformable and will absorb 14 times their weight in isotonic saline. They are soluble in 0.5N sodium hydroxide and are characterized by their carboxylic acid content which is found by titration to be 26.3%.
A solution of the oxidized carboxymethyl cellulose was prepared by dissolving the sponge material at a concentration of 10mg/ml in 0.1M ammonium hydroxide. The solution was incubated at 37°C for 2 hours, and the reaction was then stopped by the addition of 6M HCl until precipitation occurred. The precipitate was collected and dialysed extensively against distilled water in tubing with a 1000 dalton molecular weight cut off, then freeze dried to produce a powder.
The molecular weight was determined by gel electrophoresis and found to be 1000 and 30000 daltons.

Example 4

Methyl cellulose was oxidized by a procedure analogous to that described in Example 3, and a solution was prepared by dissolving the oxidized material at a concentration of 20mg/ml in 6M sodium hydroxide and incubating at 37°C for 45 minutes. The solution was centrifuged to remove any undissolved material, and the oligosaccharides were precipitated out of solution by the addition of 6M HCl. The precipitate was collected and dialysed extensively against distilled water in tubing with a 1000 dalton molecular weight cut off.
The molecular weight was determined by gel electrophoresis and found to be between 1000 and 5000 daltons.

Example 5

A phenyl boronic acid (PBA) solid phase extraction column (Bond Elut, Varian Associates), containing 100mg of sorbent material with a 10ml reservoir, was activated using 10ml of methanol to wet the column, followed by 10ml 0.1M acetic acid to equilibrate the column at the correct pH.

ORC solution was prepared by dissolving 1g of Interceed™ material in 100ml 6M sodium hydroxide solution. After the Interceed™ material had fully dissolved, the solution was acidified to pH 3.0 and any precipitate was removed by centrifugation. The supernatant was taken, and 2ml was passed through the activated PBA column. The column was then washed with 2ml 0.1M acetic acid and 4x2ml portions of distilled water to remove any salt or other endogenous material. The ORC oligosaccharides were eluted from the column using 2x2ml portions of 0.1M ammonium hydroxide. the portions were pooled, frozen and lyophilised to produce a powder. After separation of the oxidized oligosaccharides by ion-exchange chromatography, mass spectrographic analysis shows them to have a molecular weight in the range 600 to 1200 daltons.

Example 6

A collagen/ORC oligosaccharide sponge was prepared in the following way. Limed collagen fibres (0.8g) were slurried in 160ml 0.01 HCl (pH3.0), and 0.16g of ORC oligosaccharide, prepared as in Example 2, was added. The mixture was homogenised for 15 seconds, HMDI was added (10% w/w of collagen) and the slurry was homogenised for a further 2 x 15 seconds. The slurry was degassed, poured into two 9cm diameter petri dishes, frozen and freeze dried using a heat ramp running from -30° to 70°C over 72 hours.

The collagen/ORC oligosaccharide sponge was then tested for its ability to bind platelet-derived growth factor (PDGF). For comparative purposes, Interceed™ fabric and a simple collagen sponge (prepared as described above, but without the addition of ORC oligosaccharide) were also tested. In each case, a small section of test material (approximately 1cm$^2$ squares of Interceed™ fabric, and approximately 1cm x 0.5cm x 0.4cm sections of sponge) were weighed and soaked in 100mM sodium phosphate dibasic buffer containing 150mM sodium chloride (total volume 1ml) for at least one hour at room temperature. Samples were then incubated with 2% bovine serum albumin (BSA) in phosphate buffered saline (PBS) for 2 hours at room temperature. 25ng of PDGF was then added to each sample in 250µl of PBS containing 2% BSA, and samples were then incubated for a further hour at 37°C.

Each sample was then washed three times with 250µl PBS followed by increasing concentrations of sodium chloride. Finally, each sample was washed with 4.0M urea. ELISA analyses of the original PDGF preparation and the various washings from the sample materials provided the following results:

TABLE I:

| BINDING OF PDGF-AB | | | |
|---|---|---|---|
| SAMPLE | COLLAGEN | COLL/ORC | INTERCEED |
| Original | 100% | 100% | 100% |
| Unbound | 20.6% | 22.9% | 15.4% |
| PBS wash | 1.8% | 11.1% | 7.5% |
| 0.3M NaCl | 4.50% | 12.3% | 1.9% |
| 0.5M NaCl | 22.0% | 22.2% | 7.7% |
| 1.0M NaCl | 11.9% | 15.2% | 11.2% |
| 2.0M NaCl | 3.0% | 5.1% | 7.8% |
| 3.0M NaCl | 0% | 4.3% | 3.4% |
| 4.0M urea | 0% | 4.0% | 9.7% |
| Recovered | 63.8% | 97.1% | 64.6% |

The results show that the three test materials all bind similar quantities of PDGF, but PDGF can be recovered in highest yield from the collagen/ORC oligosaccharide sponge.

The binding characteristics are also uniquely different for the collagen/ORC oligosaccharide materials compared with the individual comparison materials. These observations indicate the complex has unique binding of PDGF which may be utilized appropriately for both exogenous binding and endogenous binding and release of growth factor.

**Claims**

1. A pharmaceutical composition for topical, oral or parenteral administration comprising an oxidized oligosaccharide having an average molecular weight in the range 1000 to 50000 daltons.

2. A pharmaceutical composition according to claim 1 comprising an oxidised oligosaccharide derived from an oxidized bacterial or plant polysaccharide.

3. A pharmaceutical composition according to claim 1 comprising an oxidised oligosaccharide derived from an oxidized animal polysaccharide or an oxidized synthetic polysaccharide.

4. A pharmaceutical composition according to claim 1, comprising an oxidised oligosaccharide derived from oxidized cellulose or an oxidized cellulose derivative.

5. A pharmaceutical composition according to claim 1, comprising an oxidised oligosaccharide derived from an oxidized derivative of dextran, gellan gum, xanthan gum, agar, starch, konjac, carrageenan, guar gum, pectin, carboxymethyl cellulose, methylhydroxypropyl cellulose, cellulose acetate, methyl cellulose, cellulose phosphate, ethyl cellulose, or inulin.

6. A pharmaceutical composition according to any preceding claim comprising an oxidised oligosaccharide having an average molecular weight in the range 5000 to 25000 daltons.

7. A pharmaceutical composition according to any preceding claim, wherein the oxidized oligosaccharide composition is bound to a pharmacologically active peptide or protein.

8. A pharmaceutical composition according to claim 7, wherein the peptide or protein is a growth factor.

9. Use of a pharmaceutical composition according to any of claims 1 to 8 for the preparation of a composition for use as or in a wound dressing.

10. Use according to claim 9, wherein a pharmacologically active agent is distributed substantially uniformly throughout said wound dressing.

11. Use according to claim 10 wherein said pharmacologically active agent is an antibiotic, an antiseptic or a protein growth factor.

12. A method of preparing the oxidized cellulose of claim 1, the method comprising the steps of:

   (a) treating an oxidized cellulose having an average molecular weight of at least 5000 with an aqueous alkaline solution at a temperature and for a period of time sufficient to result in partial hydrolysis of said oxidized cellulose; and
   (b) recovering the resulting oxidized cellulose from said solution.

13. A method according to claim 12 wherein the alkaline solution is a solution of an alkali metal hydroxide or bicarbonate.

14. A method according to claim 12 or claim 13 wherein the oxidized cellulose is recovered from said solution by adjusting the pH to 7 or less using an acid.

15. A method according to claim 14 wherein the acid is a concentrated mineral acid.

16. A method according to any of claims 12 to 15, the method comprising the steps of:

   (a) providing an alkaline solution of said oxidized cellulose;
   (b) dissolving or dispersing a therapeutically active agent in said alkaline solution; and
   (c) reducing the pH of said solution or dispersion to cause said oxidized cellulose to be precipitated.

17. A method according to any of claims 12 to 15, the method comprising the steps of:

(a) providing an alkaline solution of said oxidized cellulose;
(b) dissolving or dispersing a therapeutically active agent in said alkaline solution; and
(c) removing the solvent from said solution or dispersion.

**18.** A method according to claim 17 wherein the solvent is removed in step (c) by freeze-drying.


**Patentansprüche**

**1.** Pharmazeutische Zusammensetzung zur topischen, oralen oder parenteralen Verabreichung, umfassend ein oxidiertes Oligosaccharid mit einem mittleren Molekulargewicht im Bereich von 1000 bis 50.000 Dalton.

**2.** Pharmazeutische Zusammensetzung nach Anspruch 1, umfassend ein oxidiertes Oligosaccharid, das von einem oxidierten bakteriellen oder pflanzlichen Polysaccharid abgeleitet ist.

**3.** Pharmazeutische Zusammensetzung nach Anspruch 1, umfassend ein oxidiertes Oligosaccharid, das von einem oxidierten tierischen Polysaccharid oder einem oxidierten synthetischen Polysaccharid abgeleitet ist.

**4.** Pharmazeutische Zusammensetzung nach Anspruch 1, umfassend ein oxidiertes Oligosaccharid, das von oxidierter Zellulose oder einem oxidierten Zellulosederivat abgeleitet ist.

**5.** Pharmazeutische Zusammensetzung nach Anspruch 1, umfassend in oxidiertes Oligosaccharid, das von einem oxidierten Derivat von Dextran, Gellangummi, Xanthangummi, Agar, Stärke, Konjac, Carrageenan, Guargummi, Pectin, Carboxymethylzellulose, Methylhydroxypropylzellulose, Zelluloseacetat, Methylzellulose, Zellulosephosphat, Ethylzellulose oder Inulin abgeleitet ist.

**6.** Pharmazeutische Zusammensetzung nach einem der vorangehenden Ansprüche, umfassend ein oxidiertes Oligosaccharid, das ein mittleres Molekulargewicht im Bereich von 5000 bis 25.000 Dalton aufweist.

**7.** Pharmazeutische Zusammensetzung nach einem der vorangehenden Ansprüche, bei dem die Zusammensetzung oxidierten Oligosaccharids an ein pharmakologisch aktives Peptid oder Protein gebunden ist.

**8.** Pharmazeutische Zusammensetzung nach Anspruch 7, bei der das Peptid oder Protein ein Wachstumsfaktor ist.

**9.** Verwendung einer pharmazeutischen Zusammensetzung nach einem der Ansprüche 1 bis 8 zur Herstellung einer Zusammensetzung zum Gebrauch als oder für einen Wundverband.

**10.** Verwendung nach Anspruch 9, bei der ein pharmakologisch aktives Mittel im wesentlichen einheitlich innerhalb des Wundverbandes verteilt ist.

**11.** Verwendung nach Anspruch 10, bei der das pharmakologisch aktive Mittel ein Antibiotikum, ein Antiseptikum oder ein Protein-Wachstumsfaktor ist.

**12.** Verfahren zur Herstellung der oxidierten Zellulose nach Anspruch 1, wobei das Verfahren die Schritte umfaßt von:

(a) Behandeln einer oxidierten Zellulose mit einem mittleren Molekulargewicht von wenigstens 5000 mit einer wässrigen Alkalilösung bei einer Temperatur und für eine Zeitdauer, die ausreichen, um zur teilweisen Hydrolyse der oxidierten Zellulose zu führen; und
(b) Wiedergewinnen der erhaltenen oxidierten Zellulose aus der Lösung.

**13.** Verfahren nach Anspruch 12, bei dem die alkalische Lösung eine Lösung eines Alkalimetallhydroxides oder -bicarbonates ist.

**14.** Verfahren nach Anspruch 12 oder Anspruch 13, bei dem die oxierte Zellulose durch Einstellen des pH auf 7 oder weniger unter Verwendung einer Säure wiedergewonnen wird.

**15.** Verfahren nach Anspruch 14, bei dem die Säure eine konzentrierte Mineralsäure ist.

**16.** Verfahren nach einem der Ansprüche 12 bis 15, wobei das Verfahren die Schritte umfaßt von:

(a) Bereitstellen einer alkalischen Lösung der oxidierten Zellulose;
(b) Auflösen oder Dispergieren eines therapeutisch aktiven Mittels in der alkalischen Lösung; und
(c) Erniedrigen des pH der Lösung oder Dispersion um zu bewirken, daß die oxidierte Zellulose präzipitiert.

**17.** Verfahren nach einem der Ansprüche 12 bis 15, wobei das Verfahren die Schritte umfaßt von:

(a) Bereitstellen einer alkalischen Lösung der oxidierten Zellulose;
(b) Auflösen oder Dispergieren eines therapeutisch aktiven Mittels in der alkalischen Lösung; und
(c) Entfernen des Lösungsmittels aus der Lösung oder Dispersion.

**18.** Verfahren nach Anspruch 17, bei dem das Lösungsmittel in Schritt (c) durch Gefriertrocknen entfernt wird.

## Revendications

**1.** Composition pharmaceutique pour administration topique, orale ou parentérale, comprenant un oligosaccharide oxydé ayant une masse moléculaire moyenne comprise entre 1.000 et 50.000 daltons.

**2.** Composition pharmaceutique selon la revendication 1, comprenant un oligosaccharide oxydé qui dérive d'un polysaccharide bactérien ou végétal oxydé.

**3.** Composition pharmaceutique selon la revendication 1, comprenant un oligosaccharide oxydé qui dérive d'un polysaccharide animal oxydé ou d'un polysaccharide synthétique oxydé.

**4.** Composition pharmaceutique selon la revendication 1, comprenant un oligosaccharide oxydé qui dérive d'une cellulose oxydée ou d'un dérivé de la cellulose oxydée.

**5.** Composition pharmaceutique selon la revendication 1, comprenant un oligosaccharide oxydé qui dérive d'un dérivé oxydé du dextran, de la gomme gellane, de la gomme xanthane, de la gélose, de l'amidon, du konjac, du carragénane, de la gomme de guar, de la pectine, de la carboxyméthylcellulose, de la méthylhydroxypropylcellulose, de l'acétate de cellulose, de la méthylcellulose, du phosphate de cellulose, de l'éthylcellulose ou de l'inuline.

**6.** Composition pharmaceutique selon l'une quelconque des revendications précédentes, comprenant un oligosaccharide oxydé ayant une masse moléculaire moyenne comprise entre 5.000 et 25.000 daltons.

**7.** Composition pharmaceutique selon l'une quelconque des revendications précédentes, dans laquelle la composition d'oligosaccharide oxydé est liée à un peptide ou une protéine pharmacologiquement actif.

**8.** Composition pharmaceutique selon la revendication 7, dans laquelle le peptide ou la protéine est un facteur de croissance.

**9.** Utilisation d'une composition pharmaceutique selon l'une quelconque des revendications 1 à 8 pour préparer une composition destinée à être utilisée en tant que pansement ou dans un pansement.

**10.** Utilisation selon la revendication 9, dans laquelle un agent pharmacologiquement actif est réparti d'une manière essentiellement uniforme dans toute la masse dudit pansement.

**11.** Utilisation selon la revendication 10, dans laquelle ledit agent pharmacologiquement actif est un antibiotique, un antiseptique ou un facteur de croissance de protéines.

**12.** Procédé de préparation de la cellulose oxydée de la revendication 1, le procédé comprenant les étapes de :

(a) traitement d'une cellulose oxydée ayant une masse moléculaire moyenne d'au moins 5.000 avec une solution alcaline aqueuse à une température et pendant un laps de temps suffisants pour qu'il en résulte une hydrolyse partielle de ladite cellulose oxydée ; et

(b) récupération, à partir de ladite solution, de la cellulose oxydée obtenue.

**13.** Procédé selon la revendication 12, dans lequel la solution alcaline est une solution d'un hydroxyde ou d'un bicarbonate d'un métal alcalin.

**14.** Procédé selon la revendication 12 ou 13, dans lequel la cellulose oxydée est récupérée de ladite solution par ajustement du pH à 7 ou moins à l'aide d'un acide.

**15.** Procédé selon la revendication 14, dans lequel l'acide est un acide minéral concentré.

**16.** Procédé selon l'une quelconque des revendications 12 à 15, le procédé comprenant les étapes de :

(a) mise à disposition d'une solution alcaline de ladite cellulose oxydée ;

(b) dissolution ou dispersion, dans ladite solution alcaline, d'un agent thérapeutiquement actif ; et

(c) réduction du pH de ladite solution ou dispersion pour provoquer une précipitation de ladite cellulose oxydée.

**17.** Procédé selon l'une quelconque des revendications 12 à 15, le procédé comprenant les étapes de :

(a) mise à disposition d'une solution alcaline de ladite cellulose oxydée ;

(b) dissolution ou dispersion, dans ladite solution alcaline, d'un agent thérapeutiquement actif ; et

(c) élimination du solvant de ladite solution ou dispersion.

**18.** Procédé selon la revendication 17, dans lequel le solvant est éliminé dans l'étape (c) par lyophilisation.